# EUROPEAN PATENT SPECIFICATION

(11) **EP 0 429 619 B1**
(45) Date of publication and mention of the grant of the patent: **25.08.1993**
(21) Application number: 90909481.5
(22) Date of filing: 21.06.1990
(51) Int. Cl.: C07C 323/43, C07C 309/15, C07C 319/20, C07C 317/42

(54) **NEW PROCESS FOR THE PREPARATION OF PHENYL-GUANIDINE DERIVATIVES**
NEUES VERFAHREN ZUR HERSTELLUNG VON PHENYL-GUANIDIN-DERIVATEN
NOUVEAU PROCEDE DE PREPARATION DE DERIVES DE PHENYL-GUANIDINE

(30) Priority: 21.06.1989 HU 315489
(43) Date of publication of application: 05.06.1991
(73) Proprietor: CHINOIN Gyogyszer és Vegyészeti Termékek Gyára RT., H-1045 Budapest IV (HU)
(72) Inventor: HUSZAR, Csaba, H-1124 Budapest (HU); SPERBER, Ferenc, H-1144 Budapest (HU); NEMETH, Attila, H-2131 Göd (HU); SARKÖZI, Péter, H-1012 Budapest (HU); SOMFAI, Eva, H-1014 Budapest (HU); PALI, Lajosné, H-1043 Budapest (HU)
(74) Representative: Skailes, Humphrey John
(86) International application number: HU9000040
(87) International publication number: WO9015795

(56) References cited:
- EP-A- 0 050 286
- US-A- 4 406 893
- US-A- 4 723 029
- US-A- 4 781 866

## Description

The invention relates to a new process for the preparation of known phenyl-guanidine derivatives.
Due to their anthelmintic activity some guanidine derivatives are used primarily in the veterinary therapy (.EP-A 50 286). The present invention relates to the preparation of these active ingredients of the general formula (I)

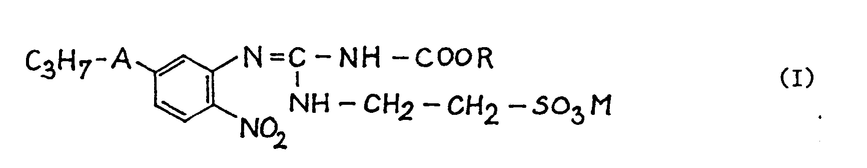

The meaning of the variable substituents in this specification is always as follows:
R is a C₁₋₄ alkyl group,
M is a hydrogen atom or a sodium, potassium or calcium ion,
A is an -S-, -SO- or -SO₂- group.

The most simple method as preparing guanidines is to react S-alkyl-isothiuronium salts with ammonia or with amines (Org. Synth. Coll. Vol. III : 440; J. Am. Soc. 55 : 1280; J. Am. Soc. 78: 6144: J. Am. Soc. 1946 : 1063; USP 4211867). In practice generally the S-methyl-isothiuronium salts are used, but their great disadvantage is that as by- product methyl-mercaptan is formed which has a very disagreeable smell and is very toxic. A further disadvantage is that the reactivity of the S-methyl group is very low and the reaction can take several days.

Guanidines can be prepared by reacting ammonia or ammonium-compounds with cyanamide (Org. Synth. 7 46**;** Ber 37 : 1681: Ber. 50 : 1620), with carbodiimide (USP 4414211, Tetrahedron 37: 233), with chloro-formamidine (Ber. 94: 2278; Ber. 97 : 1232) or with dichloro-isocyanide (USP 4211867).

The required reactants are generally corrosive and toxic and/or sensitive to humidity.

The patents protecting the preparation of phenyl-guanidines of the formula (I) are describe the reaction of N-methoxycarbonyl-N'-(2-nitro-4-propyl-thiophenyl)-S-methyl-isothiocarbamide and N'-(2-aminoethyl)-N'-cyano-N''-methyl-guanidine with a reaction time of 24 hours, using 2 molar equivalents of amine in acetonitrile (EP-A 50 286).
This process can be reproduced for compounds of the formula (I), even with a reaction time of several days, only with a yield of 45%. The probable reason is that the S-methyl group reacts very slowly by nucleophilis substitution, and the reaction leads to by-products (USP 4406983).
The present invention relates to the preparation of guanidine derivatives of the formula (I) 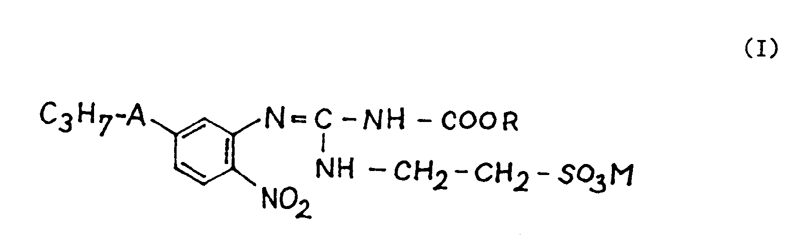
characterised in that a thiocarbamide derivative of formula (II) 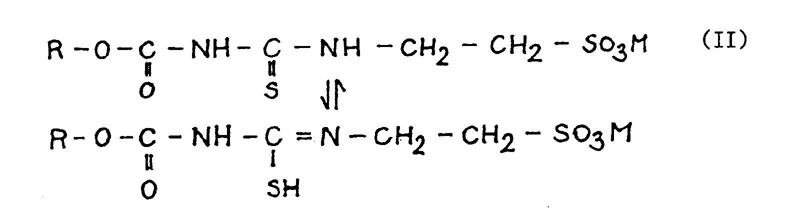
is oxidized, optionally in the presence of an inorganic or organic solvent, and the new sulfonic-acid derivative of the formula (III) 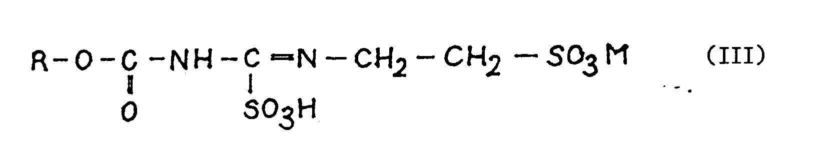
thus obtained is reacted with an aniline derivative of the formula (IV) 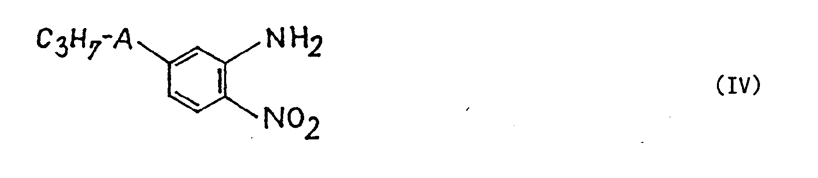
optionally in the presence of an inorganic or organic solvent.

The invention is based on the recognition that the new compound of formula (III) containing an -SO₃ group reacts essentially more quickly with derivatives of aniline of formula (IV), as suitable derivatives containing an -SH group (the reaction speed is about 15 fold greater). A further recognition is that the new compounds of the formula (III) can be prepared in a very simple way by oxidizing the -SH-derivative of formula (II).

The oxidation is carried out advantageously with oxygen, hydrogen peroxide, sodium metaperiodate, potassium permanganate, with chlorates or with some peracids, optionally in the presence of water and/or an organic solvent.

In carrying out the oxidation with hydrogen peroxide at 0-45 °C, water can be used as solvent. In this reaction advantageously a catalyst is used, e.g. sodium molybdate in catalytic quantity.

The oxidation according to the invention can be realized by using, as the organic peracid, peracetic acid, per-benzoic acid, m-chloro-perbenzoic acid or perphthalic acid.

The sulfonic acid derivative of formula (III) can be reacted in the presence of acetonitrile, dimethylformamide, dimethylacetamide, dioxane and/or dimethylsulfoxide with the aniline derivative of formula (IV) at temperatures between 0°C and the boiling point of the reaction mixture.

The amine of the general formula (IV) is advantageously used in an excess of 5-30%.

The advantage of the method according to the invention is that it is simple and leads not to smelly or toxic by -products and the isolated intermediates are stable at room temperature. The process has generally a short reaction time and gives a good yield (above 70%).
Compounds of formulae (II) and (IV) are known in the art and may be prepared by methods well-known to those skilled in the art.

The process according to the invention is illustrated in detail by the following non limiting Examples.

### Example 1

31,33 g of N-(methoxycarbonyl)-N'-(2-ethylsulfonic acid)-thiocarbamide are suspended in 100ml of water, 3 g of sodium chloride and 0,5 g of sodium molybdate are added and cooled to 0°C. Thereafter 43 ml of 33% hydrogen peroxide solution is added dropwise, in a way that 28 ml are added below 20°C and the remainder below 40°C. The duration of the addition is about 1 hour. The mixture is cooled to 10°C, filtered and the residue is washed with cold, saturated sodium chloride solution and dried.
The product obtained is added to 32 g of 2-nitro-4-propylthioaniline in 100 ml of acetonitrile and the mixture is refluxed until the starting sulfonic acid disappears (thin layer chrom.: solide phase : Polygram Sil G/UV₂₅₄; eluting mixture: methanol, acetic acid, chloroform 95:5:5). The solvent is distilled in vacuo. To the residue 10 g of sodium hydroxide dissolved in 100 ml of water are added, the solution is filtered with a small quantity of charcoal and the pH value of the filtrate is adjusted to 1,5. The filtered crystals are washed with acetone. 42,6 g of N-(methoxycarbonyl)-N'-((2-nitro-5-propylsulfenyl)phenyl)-N''-(2-ethylsulfonic acid) guanidine are obtained. Mp.: 214-215 °C.

### Example 2

2,41 g of N-(methoxycarbonyl)-N'-(2-ethylsulfonic-acid)-thiocarbamide are suspended in 30 ml of ether. At a temperature of 20°C 5,5 g of m-chloro-per-benzoic acid dissolved in 20 ml of ether are added. After 10 minutes the solution obtained is admixed with 3x50 ml of a 5% sodium hydrogen carbonate solution, dried on magnesium sulfate and evaporated. 2,5 g of N-(methoxycarbonyl)-N'-(2-ethylsulfonic acid) amino-imino-methanesulfonic acid are obtained. Mp.: 211-216 °C.

### Example 3

5 g of N-(methoxycarbonyl)-N'-(2-ethylsulfonic-acid)-thiocarbamide are suspended in 100 ml of acetic acid and at 10°C 6 ml of a 30% hydrogen peroxide solution are added dropwise. The mixture is stirred for 6 hours at 20°C. Thereafter it is evaporated to dryness. The residue is suspended in acetone and filtered and dried. 5,1g of N-(methoxy-carbonyl)-N'-/2-ethyl-sulfonic acid)-amino-imino-methansulfonic acid are obtained. Mp.: 213-214 °C.
To the product above, 5,5 g of 2-nitro-4-propylsulfinyl-aniline and 50 ml of dimethylformamide are added. The solution is stirred until the starting sulfonic acid can not be detected by thin layer chromatography. Thereafter the solution is evaporated to dryness in vacuo and to the residue 50 ml of acetone are added. After further stirring the crystals are filtered.
6,8 g (81%) of N-(methoxycarbonyl)-N'-(2-nitro-5-propyl-sulfinyl)phenyl-N''-(2-ethylsulfonic acid)-guanidine are obtained. Mp.: 227-231 °C.

### Example 4

2,4 g N-(methoxycarbonyl)-N'-(2-ethylsulfonic acid)-thiocarbamide are suspended in 50 ml of acetic acid. At 0°C 2,3 g of peracetic acid dissolved in 20 ml of acetic acid are added. The mixture is stirred for 2 hours at room temperature and thereafter it is evaporated in vacuo to dryness. To the residue, 50 ml of dimethyl-acetamide and 3,2 g of 2-nitro-4-propyl-sulfonyl-aniline are added. The mixture is refluxed until sulfonic acid can not be detected by thin layer chromatography. Then it is evaporated in vacuo to dryness, the residue is stirred in 50 ml of acetone, filtered and the crystals obtained are washed with acetone.
3,75 g of N-(methoxycarbonyl)-N'-(2-nitro-5-propyl-sulfonyl)phenyl-N''-(2-ethylsulfonic acid)-guanidine are obtained. Mp.: 240-245 °C.

### Example 5

4,2 g of N-(methoxycarbonyl)-N'-((2-nitro-5-propyl-sulfenyl))phenyl)-N''-(2-ethylsulfonic acid)-guanidine are dissolved in the solution of 0,42 g of sodium hydroxide in 80 ml of water. A solution of 2,7 g of potassium peroxide-sulfate in 10 ml of water is added. The reaction mixture is stirred for 3 hours, and then evaporated to dryness in vacuo. The residue is suspended in 100 ml of hot methyl alcohol and then cooled to 20 °C. The precipitated crystals are filtered and washed with methyl alcohol. 3,96 g of N-(methoxycarbonyl)-N'-(2-nitro-5-propyl-sulfinyl)-phenyl-N''-(2-ethylsulfonic acid)-guanidine sodium salt are obtained. Mp.: 147-150 °C.

### Example 6

4,2 g of N-(methoxycarbonyl)-N''-(2-nitro-5-propyl-sulfenyl)-phenyl(N''-(2-ethylsulfonic acid)-guanidine are dissolved in a solution of 0,42 g of sodium hydroxide in 80 ml of water. To the solution at 20 °C, 6 ml of a 33% hydrogen peroxide solution is dropwise added. After stirring for 8 hours a solution of 2,2 g of calcium chloride in 10 ml of water is added. After stirring for 20 minutes the crystals obtained are filtered, washed with cold water and dried.
3,97 g of N-(methoxycarbonyl)-N''-((2-nitro-5-propyl-sulfonyl)-phenyl)-N''-(2-ethylsulfonic acid)-guanidine calcium salt are obtained. Mp.: 330 °C.

### Example 7

To 1 g of N-(methoxycarbonyl)-N'-(2-ethylsulfonic acid)amino-imino-methane sulfonic acid prepared according to example 2,1 g of 2-nitro-5-propylsulfinylaniline and 5 ml of acetonitrile are added. The mixture is refluxed until the starting sulfonic acid can not be detected by thin layer chromatography. Thereafter it is evaporated to dryness and to the residue 20 ml of a 1 N sodium hydroxide solution are added. The solution is clarified with charcoal and the pH value of the filtered solution is adjusted with hydrochloric acid to 1.5. 1,62 g of N-(methoxycarbonyl)-N'-(2-nitro-5-propylsulfinyl)phenyl-N''(2-ethylsulfonic acid guanidine are obtained. Mp.: 146-148 °C.

### Example 8

To 1 g of N-(methoxycarbonyl)-N'-(2-ethylsulfonic acid)-amino-imino-methane sulfonic acid 1 g of 2-nitro-5-propylsulfonylaniline and 10 ml of acetonitrile are added. The mixture is refluxed until the starting sulfonic acid can not be detected by thin layer chromatography. Thereafter it is evaporated to dryness and the residue is taken up in 10 ml of a 1 N sodium hydroxide solution. The solution is clarified with charcoal and the pH value of the filtrate is adjusted to 1.5. The precipitate thus obtained is filtered.
1,39 g of N-(methoxycarbonyl)-N'-(2-nitro-4-propylsulfonyl)phenyl-N''-(2-ethylsulfonic acid)-guanidine are obtained. Mp.: 241-244°C.

## Claims

1. Process for the preparation of guanidine derivatives of the general formula (I), 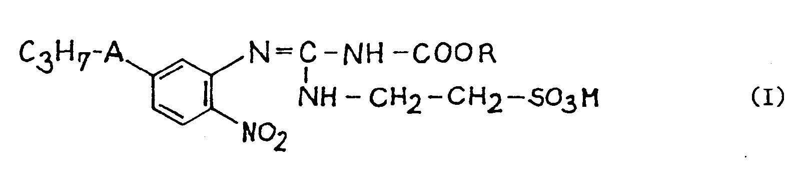 wherein
R is a C₁₋₄ alkyl group,
M is a hydrogen atom, or a sodium, potassium or calcium ion, and
A is an -S-, -SO-, or -SO₂- group-
characterized in that thiocarbamide derivative of the formula (II), 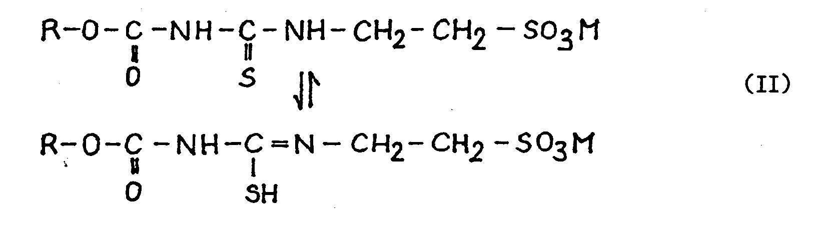 (wherein R and M have the same meanings as above) is oxidized, optionally in the presence of an inorganic or organic solvent, and the resulting sulfonic acid derivative of formula (III) 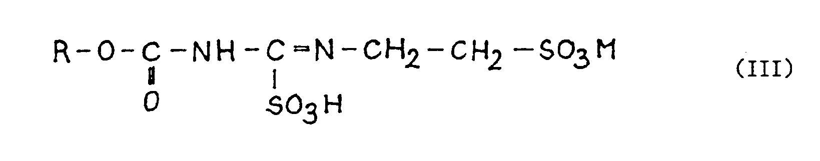 (wherein the meanings of R and M are the same as above) is reacted with an aniline derivative of the formula (IV) 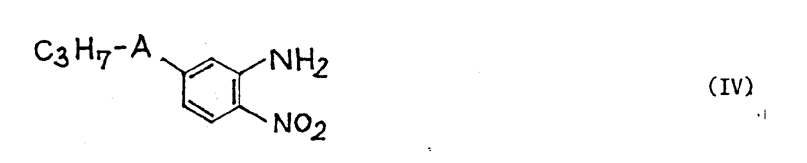 (where the meaning of A is the same as above), optionally in the presence of an inorganic or organic solvent.

2. Process according to claim 1, characterized in that the oxidation is carried out with hydrogen peroxide, sodium meta-periodate or with an organic peracid, optionally in the presence of water and a catalytic quantity of sodium molybdate.

3. Process according to claim 2, characterized in that the oxidation is carried out with hydrogen peroxide in the presence of water and a catalytic quantity of sodium molybdate.

4. Process according to claim 2, characterized in that, as organic peracid, peracetic acid, perbenzoic acid, m-chloroperbenzoic acid or perphthalic acid is used.

5. Process according to any of claims 1-4, characterized in that the sulfonic acid derivative of the formula (III) is reacted in the presence of dimethylformamide, dimethyl-acetamide, dioxane and/or dimethylsulfoxide with an aniline derivative of the formula (IV) at temperatures between 0°C and the boiling point of the reaction mixture.

6. New sulfonic acid derivatives of the formula (III) 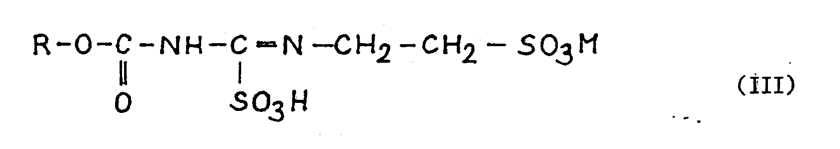 wherein
R is a C₁₋₄ alkyl group,
M is a hydrogen atom or a sodium, potassium, or calcium ion.

## Patentansprüche

1. Verfahren zur Herstellung von Guanidin-derivaten der allgemeinen Formel I: 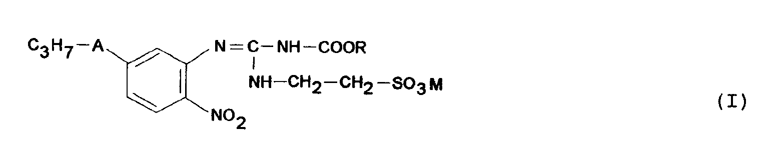 worin
R eine C₁₋₄-Alkylgruppe
M ein Wasserstoffatom oder ein Natrium-, Kalium- oder Calcium-ion und
A eine -S-, -SO- oder -SO₂- Gruppe bedeuten,
dadurch gekennzeichnet, daß ein Thiocarbamid-derivat der Formel II: 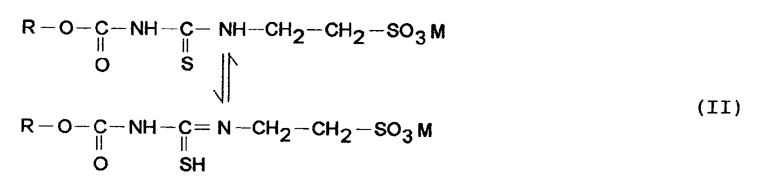 worin R und M die oben angegebene Bedeutung haben,
gegebenenfalls in Gegenwart eines organischen oder anorganischen Lösungsmittels, oxidiert wird, und das enthaltene Sulfonsäurederivat der Formel III: 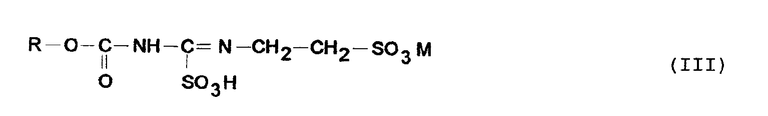 worin R und M die oben angegebene Bedeutung haben,
mit einem Anilin-derivat der Formel IV: 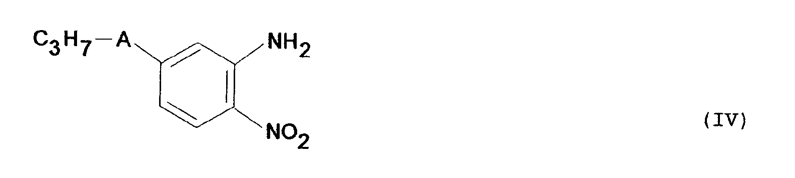 worin A die oben angegebene Bedeutung hat,
gegebenenfalls in Gegenwart eines organischen oder anorganischen Lösungsmittels umgesetzt wird.

2. Verfahren nach Anspruch 1, dadurch gekennzeichnet, daß die Oxydation mit Wasserstoffperoxid, Natriummetaperjodat oder mit einer organischen Persäure, gegebenenfalls in Gegenwart von Wasser und einer katalytischen Menge von Natrium-molybdat durchgeführt wird.

3. Verfahren nach Anspruch 2, dadurch gekennzeichnet, daß die Oxydation mit Wasserstoffperoxid in Gegenwart von Wasser und einer katalytischen Menge von Natrium-molybdat durchgeführt wird.

4. Verfahren nach Anspruch 2, dadurch gekennzeichnet, daß als organische Persäure, Peressigsäure, Perbenzoesäure, m-Chlorperbenzoesäure oder Perphthalsäure verwendet wird.

5. Verfahren nach einem der Ansprüche 1-4, dadurch gekennzeichnet, daß das Sulfonsäure-derivat der Formel III in Gegenwart von Diemethylformamid, Dimethylacetamid, Dioxan und/oder Dimethylsulfoxid mit einem Anilin-derivat der Formel IV bei Temperaturen zwischen 0°C und dem Siedepunkt der Reaktionsmischung umgesetzt wird.

6. Neue Sulfonsäure-derivate der Formel III: 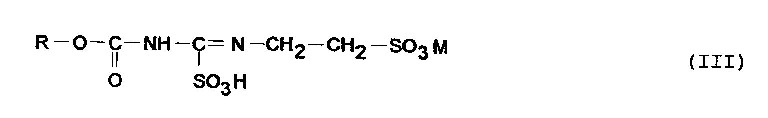 worin
R eine C₁₋₄-Alkylgruppe und
M ein Wasserstoffatom oder ein Natrium-, Kalium- oder Calciumion bedeuten.

## Revendications

1. Procédé de préparation de dérivés de guanidine de formule générale (I) 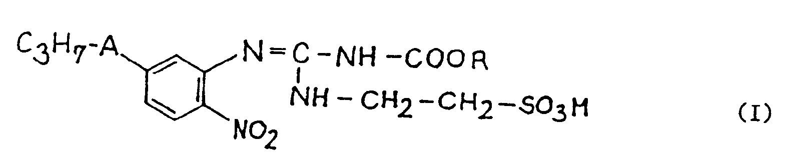 dans laquelle
R est un groupe alkyle en C₁-C₄,
M est un atome d'hydrogène ou un ion sodium, potassium ou calcium, et
A est un groupe -S-, -SO- ou -SO₂-,
caractérisé en ce que l'on oxyde un dérivé de thiocarbamide de formule (II) 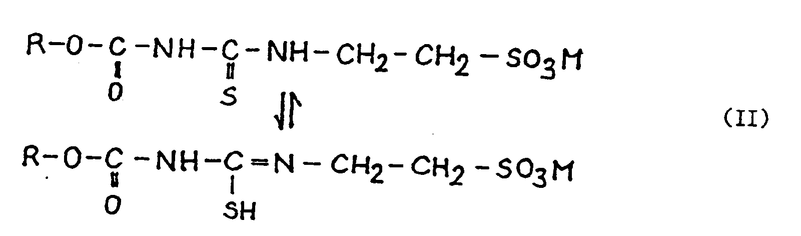 (dans laquelle R et M ont les mêmes significations que ci-dessus) éventuellement en présence d'un solvant minéral ou organique, et on fait réagir le dérivé d'acide sulfonique de formule (III) obtenu 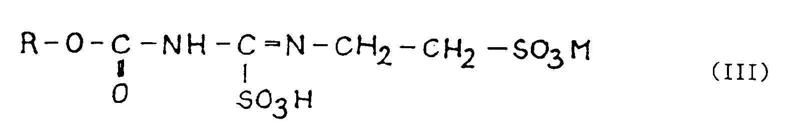 (dans laquelle R et M ont les mêmes significations que ci-dessus ) avec un dérivé d'aniline de formule (IV) 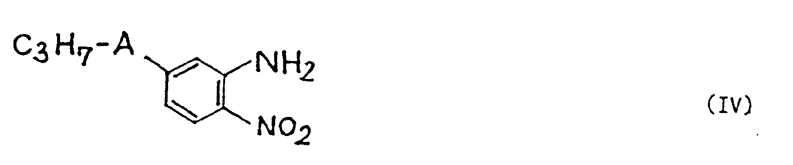 (dans laquelle A a la même signification que ci-dessus), éventuellement en présence d'un solvant minéral ou organique.

2. Procédé selon la revendication 1, caractérisé en ce que l'on effectue l'oxydation avec du peroxyde d'hydrogène, du méta-periodate de sodium ou avec un peracide organique, éventuellement en présence d'eau et d'une quantité catalytique de molybdate de sodium.

3. Procédé selon la revendication 2, caractérisé en ce que l'oxydation est réalisée avec du peroxyde d'hydrogène, en présence d'eau et d'une quantité catalytique de molybdate de sodium.

4. Procédé selon la revendication 2, caractérisé en ce que l'on utilise, comme peracide organique, l'acide peracétique, l'acide perbenzoïque, l'acide m-chloro-perbenzoïque ou l'acide perphtalique.

5. Procédé selon l'une quelconque des revendications 1-4, caractérisé en ce que l'on fait réagir le dérivé acide sulfonique de formule (III), en présence de diméthylformamide, de diméthylacétamide, de dioxane et/ou de diméthylsulfoxyde, avec un dérivé d'aniline de formule (IV), à des températures comprises entre 0°C et le point d'ébullition du mélange réactionnel.

6. Nouveaux dérivés d'acide sulfonique de formule (III) 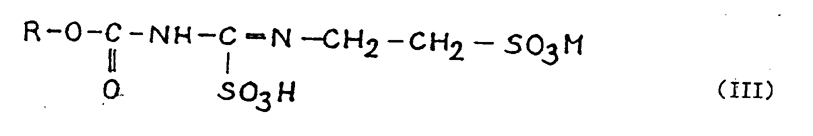 dans laquelle
R est un groupe alkyle en C₁-C₄,
M est un atome d'hydrogène ou un ion sodium, potassium ou calcium.
